# EUROPEAN PATENT SPECIFICATION

(11) **EP 3 233 027 B1**
(45) Date of publication and mention of the grant of the patent: **13.10.2021**
(21) Application number: 15798481.6
(22) Date of filing: 25.11.2015
(51) Int. Cl.: A61K 8/31, A61K 8/33, A61K 8/34, A61K 8/73, A61Q 17/04

(54) **COMPOSITIONS FOR PROVIDING IMPROVED SUNSCREEN PROTECTION**
ZUSAMMENSETZUNGEN ZUR BEREITSTELLUNG VON VERBESSERTEM SCHUTZ DURCH SONNENCREME
COMPOSITIONS POUR FOURNIR UNE PROTECTION SOLAIRE AMÉLIORÉE

(30) Priority: 15.12.2014 EP 14197880
(43) Date of publication of application: 25.10.2017
(73) Proprietor: Unilever IP Holdings B.V., 3013 AL Rotterdam (NL); Unilever Global IP Limited, Wirral, CH62 AZD (GB)
(72) Inventor: AU, Van, Trumbull, Connecticut 06611 (US); MADISON, Stephen, Alan, Trumbull, Connecticut 06611 (US)
(74) Representative: James, Helen Sarah
(86) International application number: PCT/EP2015/077619
(87) International publication number: WO 2016/096344

(56) References cited:
- WO-A1-99/33439
- WO-A2-2014/015879
- CN-A- 102 657 589
- US-A1- 2002 025 301
- US-A1- 2009 280 149

## Description

### Field of the invention

The present invention is directed to a composition for providing improved sunscreen protection. More particularly, the invention is directed to a composition comprising polymer of D-glucose and/or a non-ionic, branched heteroglycan, a sunscreen and optionally a specific alkane/alkoxy diol. The composition, upon application to skin, unexpectedly results in improved sun protection factor (SPF) as well as improved UVA protection factor (UVA-PF) to further enhance sun protection capabilities of the composition.

### Background of the invention

Ultraviolet radiation can be damaging to skin. Immediate damage may be in the form of erythema. More long term is the concern of initiating cancerous growth. For these reasons, photoprotective agents known as sunscreens have been incorporated into cosmetic products.

Organic sunscreen agents have a number of disadvantages. Under the influence of ultraviolet radiation, the sunscreen agents themselves are known to degrade. Photostability may only be a matter of hours. Consumers thinking that they are fully protected with their sunscreen application, often expose themselves for a time beyond the photostability limit. Further, organic sunscreen agents can under certain circumstances cause skin irritation. A still further problem is that some sunscreen agents may not be fully compatible with other sunscreen or formulation components.

Attempts have been made to address the aforementioned problems. One approach has been to encapsulate the sunscreen agent. A few encapsulates are commercially available.

A first commercial material is known as Sun Caps 664® sold by Particle Sciences, Inc. of Bethlehem, PA. Sun Caps 664® is formulated with a concentration of octylmethoxycinnamate (OMC) of 21.5% encapsulated in a binder that includes beeswax, carnauba wax, Vinyl Pyrrolidone/Eicosene Copolymer and emulsifiers (PEG-100 stearate, PEG-20, bis-PEG-12 dimethicone, sorbitan tristearate and Steareth-100). Sun Caps® are formed in a process revealed in U.S. Patent No. 5,733,531. The encapsulates are supplied as an aqueous dispersion containing up to 65% solids.

Another hydrophilic composite particulate commercially available is sold by Rona Division of EMD Chemicals under the trademark Eusolex® UV Pearls® OMC. UV Pearls® OMC is prepared and described in U.S. Patent No. 7,264,795. This material is delivered as 40% particles in 60% aqueous carrier. The particles are structured with a core of greater than 70% octylmethoxycinnamate surrounded by a coating of about 10% silica, about 1-2% polyvinylpyrrolidone (as binder), and minor ingredient.

While the known encapsulates have shown some advantages, formulations with the same require aggressive application to ensure adequate protection. Thus, much improvement remains to be done with respect to enhanced activity of compositions that provide sunscreen protection.

This invention, therefore, is directed to a composition that imparts improved sunscreen protection upon application to skin of a consumer. The composition comprises a polymer of D-glucose and/or a non-ionic, branched heteroglycan, and sunscreen, and unexpectedly, results in improved sun protection factor as well as improved UVA protection factor.

Document WO 2014/015879 discloses compositions comprising 0,01 to 0,2 % of thickening agents to enhance the sun protection factor of a sunscreen.

In the present invention, the problem of enhancing or improving SPF and UVA-PF has been unexpectedly achieved by formulating sunscreens in topical compositions with a polymer of D-glucose and/or a non-ionic and branched heteroglycan. Such an improvement is further enhanced when alkane diols comprising viscinally substituted hydroxy groups are included in the compositions consistent with this invention. The results that demonstrate the improved SPF and UVA-PF are based on a comparison of conventional compositions with sunscreens and compositions with the same sunscreens but made consistent with this invention.

### Summary of the invention

In a first aspect, the present invention is directed to a composition including but not limited to:
(a) about 0.5 to 7 wt. % of polysaccharide(s) selected from a water soluble and/or water dispersible nonionic polymer(s) of D-glucose, water soluble nonionic, branched heteroglycan(s) or a blend thereof;
(b) about 0.1 to 30 wt. % of a sunscreen;
(c) about 0.1 to 10 wt. % of C3 to C16 alkane, mono alkoxy or polyalkoxy diol(s) or a blend thereof, optionally wherein the diol has vicinally substituted hydroxyl groups;
(d) wherein the ratio of polysaccharide(s) to alkane/alkoxy diol(s) is in the range of about 70;1 to 1:40; and
(e) a cosmetically acceptable carrier, wherein the alkane diol includes 1,2-octane diol.

Water soluble is defined as a minimum solubility limit in water of at least 0.1 gm/liter, preferably at least 0.2, 0.5 or 1.0 gm/liter at 20 C.

In a further aspect of the invention is a composition including but not limited to:
(a) about 0.5 to 7 wt. % of polysaccharide(s) selected from locust bean gum, tara gum, pullulan, beta oat glucan, xilogel, larch arabinogalactane or a blend thereof;
(b) about 0.1 to 30 wt. % of a sunscreen; and
(e) a cosmetically acceptable carrier, wherein the sunscreen is a mixture of two or more of octylmethoxycinnamate, ethylhexyl salicylate, phenylbenzimidazole sulfonic acid, ethylhexyl p-methoxycinnamate, octocrylene and butyl methoxydibenzoylmethane.

In another aspect of the invention is a method for protecting skin from the sun including the step of contacting the skin with the above compositions.

In another aspect of the invention is the use of the above compositions to improve the efficacy of a sunscreen.

All other aspects of the present invention will more readily become apparent upon considering the detailed description and examples which follow.

Skin, as used herein, is meant to include skin on the face, neck, chest, back, arms (including underarms), hands, legs, buttocks and scalp. Polymer of D-glucose means a polymer having a glucan based backbone with alpha and/or beta 1,3-, 1,4- and/or 1,6-glycosidic linkages. Heteroglycan, as used herein, means a component comprising distinct saccharides linked by ether linkages in its backbone.

Composition, as used herein, is meant to include a topical composition that may be topically applied to the skin of a consumer in order to provide a benefit. Such a composition can be leave-on or rinse-off (like a personal wash liquid or bar, shampoo or conditioner) but is preferably, leave-on. Preferred leave-on compositions include serums, creams, lotions, balms, deodorants (including soft solids, sticks and roll-ons) and gels. Alkane diol with vicinally substituted hydroxyl groups includes C₆ to C₁₂ alkane diols, and preferably, includes 1,2-octanediol (i.e., caprylyl glycol). Mannose group means a unit or radical derived from mannose, and galactose group means a unit or radical derived from galactose. Non-ionic, branched heteroglycan may be used interchangeably with heteroglycan. Molecular weight, as used herein, means number average molecular weight (Mn) as determined by size exclusion chromatography.

SPF, as used herein, means sun protection factor which is a measure of UVB protection (sun ultraviolet radiation, 290 to 320 nm). SPF is measured by determining the amount of redness in sunscreen protected skin divided by the amount of light that causes redness in unprotected skin. UVA-PF is measured similar to SPF but is a measure of UVA protection (sun ultraviolet radiation, 320 to 400 nm).

Comprising, as used herein, is meant to include consisting essentially of and consisting of. For the avoidance of doubt, and by way of example, the composition of this invention may consist essentially of sunscreen, non-ionic, branched heteroglycan, emotives and carrier or may consist of the same. All ranges identified herein are meant to include all ranges subsumed therein if, for example, reference to the same is not explicitly made. Weight percents and molecular weights presented herein are meant to be modified by the word "about" if, in fact, such modification is not explicitly made.

### Detailed description of the invention

In a first aspect, the present invention is directed to a composition including but not limited to:
(a) about 0.5 to 7 wt. % of polysaccharide(s) selected from a water soluble and/or water dispersible nonionic polymer(s) of D-glucose, water soluble nonionic, branched heteroglycan(s) or a blend thereof;
(b) about 0.1 to 30 wt. % of a sunscreen;
(c) about 0.1 to 10 wt. % of C3 to C16 alkane, mono alkoxy or polyalkoxy diol(s) or a blend thereof, optionally wherein the diol has vicinally substituted hydroxyl groups;
(d) wherein the ratio of polysaccharide(s) to alkane/alkoxy diol(s) is in the range of about 70;1 to 1:40; and
(e) a cosmetically acceptable carrier, wherein the alkane diol includes 1,2-octane diol.

Preferably the composition contains less than 5, 4, 3, 2, 1, 0.5, 0.2 or 0.1 wt. % a water insoluble hydrophobic or hydrophobically modified polysaccharide(s) such as starch, chitosan, dextran, cyclodextrin, cellulose and hydrophobically modified pullulan, blends thereof and the like. Water insoluble is defined as a maximum solubility in water of less than 0.1 gm/liter, preferably less than 0.05 or 0.01 gm/liter at 20 C.

Preferably the polysaccharide(s) includes a polymer of D-glucose with a number average molecular weight from 50,000 to 4,500,000 (preferably derived from barley, oat, wheat, rye, tamarind gum and/or Shitake mushrooms). More preferably the polysaccharide(s) includes a nonionic, branched heteroglycan and comprises backbone groups represented as having repeat units of I and II, or II 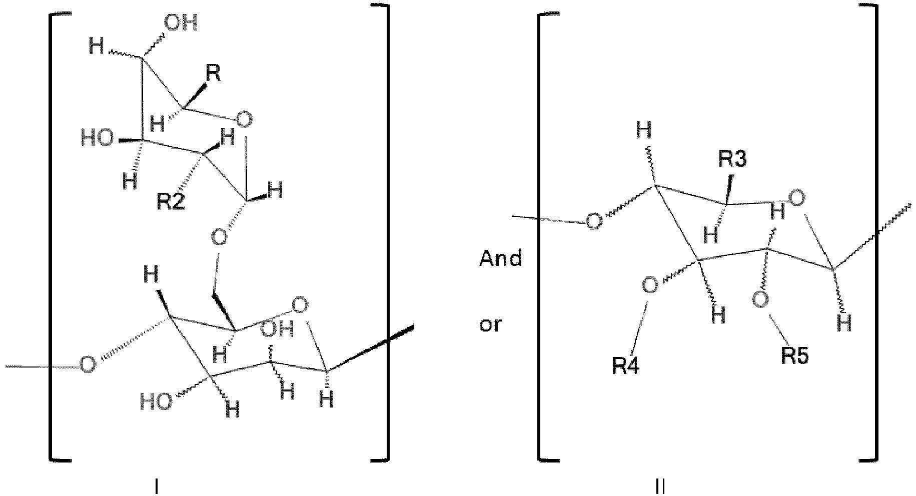 wherein
R= H, or CH₂OH;
R2= OH, or C₆ monosaccharide;
R3= H, CH₂OH, or acetate;
R4= H, or [C6 monosaccharide]ₙ n= integer from approximately 200 to 25,000;
R5= H, or C₅ monosaccharide;
and/or agar, dextran, pullulan, arabinogalactane or blends thereof.

Advantageously the non-ionic, branched heteroglycan comprises backbone groups that are at least 85% mannose group and further wherein the heteroglycan comprises branched groups that are at least 95% galactose group. Preferably the non-ionic, branched heteroglycan is derived from guar, tara, fenugreek, locust bean or carob gum or a mixture thereof.

Preferably the sunscreen is octylmethoxycinnamate, ethylhexyl salicylate, phenylbenzimidazole sulfonic acid, ethylhexyl p-methoxycinnamate, butyl methoxydibenzoylmethane, benzophenone-3, titanium dioxide, zinc oxide, p-aminobenzoic acid, octyldimethyl-PABA, 2-ethoxyethyl p-methoxy cinnamate, benzophenone-1, benzophenone-2, benzophenone-6, benzophenone-8, benzophenone-9, benzophenone-12, homomethyl salicylate, menthyl anthranilate, benzophenone-4, triethanolamine salicylate, terephthalylidene dicamphor sulfonic acid, bisoctriazole, bisethylhexyloxyphenol methoxyphenyl triazine, bisdisulizole disodium, diometriazole trisiloxane, octyltriazone, iscotrizinol, polysilicone-15, isopentenyl-4-methoxycinnamate, octocrylene or a mixture thereof. More preferably the sunscreen is octylmethoxycinnamate, ethylhexyl salicylate, phenylbenzimidazole sulfonic acid, ethylhexyl p-methoxycinnamate, octocrylene, butyl methoxydibenzoylmethane or a mixture thereof.

In a preferred embodiment the inventive composition imparts an improved SPF and UVA-PF upon use compared to the same composition absent about 0.25 to 7 wt. % of polysaccharide(s) selected from a water soluble or dispersible nonionic polymer(s) of D-glucose, water soluble or dispersible nonionic, branched heteroglycan(s) or a blend thereof.

In another aspect of the invention is a method for protecting skin from the sun comprising the step of contacting the skin with the above composition.

In a further aspect of the invention is a composition including but not limited to:
(a) about 0.5 to 7 wt. % of polysaccharide(s) selected from locust bean gum, tara gum, pullulan, beta oat glucan, xilogel, larch arabinogalactane or a blend thereof;
(b) about 0.1 to 30 wt. % of a sunscreen; and
(e) a cosmetically acceptable carrier, wherein the sunscreen is a mixture of two or more of octylmethoxycinnamate, ethylhexyl salicylate, phenylbenzimidazole sulfonic acid, ethylhexyl p-methoxycinnamate, octocrylene and butyl methoxydibenzoylmethane.

In a further aspect of the invention, the above composition imparts an improved SPF and UVA-PF upon use compared to the same composition absent about 0.25 to 7 wt. % of polysaccharide(s) selected from a water soluble nonionic polymer(s) of D-glucose, water soluble nonionic, branched heteroglycan(s) or a blend thereof.

In another aspect of the invention is a method for protecting skin from the sun comprising the step of contacting the skin with the above described compositions.

In a further aspect of the invention is the use of the above described compositions to improve the efficacy of a sunscreen.

### Polysaccharides

The only limitation with respect to the polymers of D-glucose suitable for use in this invention is that the same can be used in compositions topically applied to consumers. Such polymers are those which preferably comprise 1,3-, 1,4- and/or 1,6- glycosidic linkages. Most preferably, the polymers of D-glucose suitable for use are those comprising beta 1 3- and 1,4- glycosidic linkages at a ratio of 20:80 to 80:20, and preferably, at a ratio of 30:70 to 70:30, and most preferably, at a ratio of 45:55 to 55:45, including all ratios subsumed therein.

Especially, preferred polymers of D-glucose suitable for use are those recovered from Shitake mushrooms, as well as beta 1, 3- and 1, 4- glucans like barley, oat, wheat and rye glucan; and most especially, beta 1, 3- and 1, 4- oat glucan. These polymers are commercially available and typically sold by suppliers like Acetar Bio-Tech, Inc. and Shanghai Passiono International Co., Ltd.

Regarding the non-ionic and branched heteroglycan that may be used in this invention, the same is linked to one suitable for use in compositions that are topically applied to consumers.

The above described non-ionic and branched heteroglycan may be generally classified as a galactosemannan wherein the same is made up of galactose and mannose groups. Figures I and II represent a mannose group with a branched galactose group and a mannose group, respectively. The backbone, therefore, of the heteroglycan suitable for use in this invention is at least 95%, and preferably, 100% mannose group. Typically, groups represented by I and II are randomly dispersed in the heteroglycan. Often, the heteroglycan comprises as a branched group at least 95%, and preferably, 100% galactose group. The heteroglycan suitable for use herein often comprises at least 45% by weight mannose group, and preferably, from 45% to about 85%, and most preferably, from about 50% to 80% by weight mannose group based on total weight of the heteroglycan.

As to the galactose group, the same typically makes up at least 15% by weight of the heteroglycan, and preferably, from 15% to 55%, and most preferably, 15 to 50% by weight of the heteroglycan.

The heteroglycan used herein comprises (but preferably consists essentially of) beta 1,4-glycosideic linkages in its backbone, and comprises (but preferably consists essentially of) alpha 1,4-glycosidic linkages in its side chains. The same is made commercially available from suppliers like CP Kelco and Gum Technology Corporation.

The polymer of D-glucose and the non-ionic,branched heteroglycan used in this invention will typically have a number average molecular weight (Mn) from 50,000 to about 4,500,000, and preferably, from about 650,000 to 3,750,000, and most preferably, from about 1,000,000 to about 3,250,000.

When used alone or collectively, the polymer of D-glucose and the heteroglycan makes up from 0.5 to about 7%, and preferably, from about 0.5 to about 5%, and most preferably, from about 0.5 to about 3% by weight of the composition, based on total weight of the composition comprising the polymer of D-glucose and/or heteroglycan.

In an especially preferred embodiment, the non-ionic, branched heteroglycan used in this invention is guar (mannose: galactose ratio of approx. 2:1), tara gum (mannose: galactose ratio of approx. 3:1), fenugreek gum (mannose: galactose ratio of approx. 1:1), locust bean or carob gum (mannose: galactose ratio of approx. 4:1) or a mixture thereof. Other useful polymers of D-glucose and/or non-ionic, branched heteroglycans include Konjac glucomannan, carageenans, agar and the following:

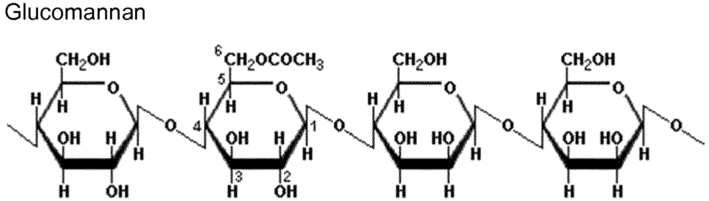

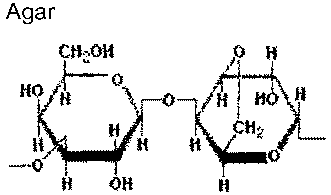

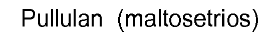

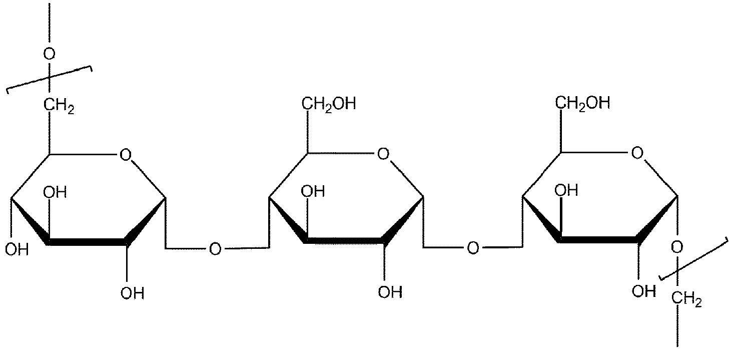

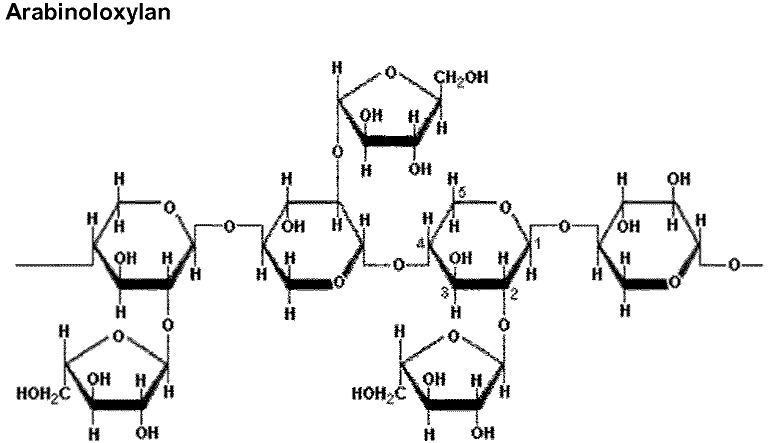

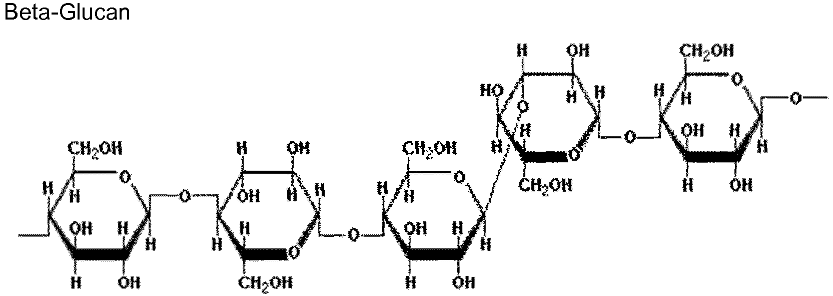

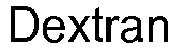

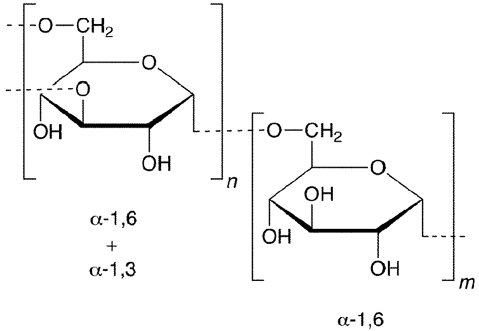

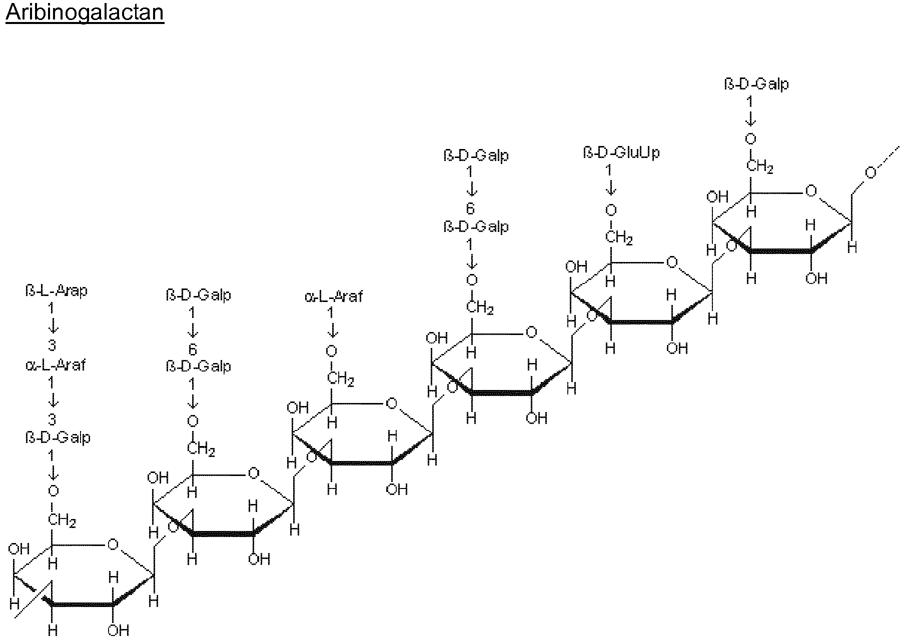

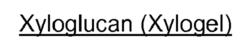

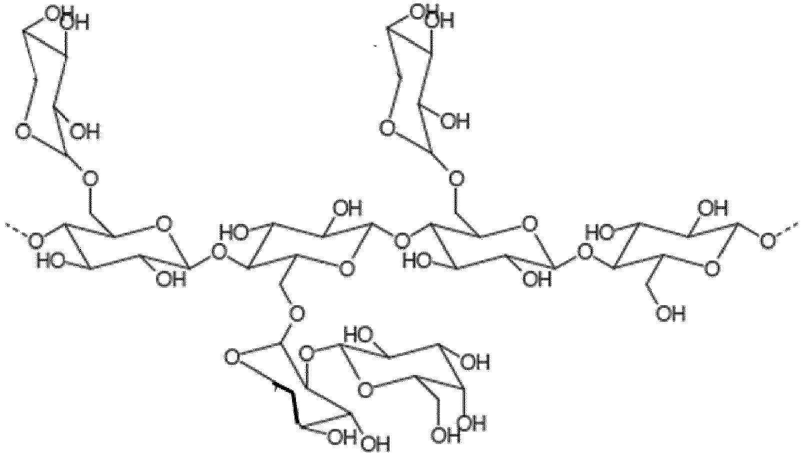

### Sunscreens:

The sunscreens that may be used in compositions of the present invention include such materials as octylmethoxycinnamate (OMK), ethylhexyl salicylate, phenylbenzimidazole sulfonic acid (Ensulizole), ethylhexyl p-methoxycinnamate, available as Parsol MCX®, Avobenzene (butyl methoxydibenzoylmethane), available as Parsol 1789® and benzophenone-3, also known as Oxybenzone. Inorganic sunscreen actives may be employed such as microfine titanium dioxide (preferably with a particle diameter of less than 150nm, and most preferably, less than 100nm) and zinc oxide may be used, polyethylene and various other polymers are also suitable sunscreens. Other sunscreens suitable for use include p-aminobenzoic acid (PABA), octyldimethyl-PABA, 2-ethoxyethyl p-methoxy cinnamate, benzophenone-1, benzophenone-2, benzophenone-6, benzophenone-8, benzophenone-9, benzophenone-12, homomethyl salicylate, menthyl anthranilate, benzophenone-4, triethanolamine salicylate, terephthalylidene dicamphor sulfonic acid, bisoctriazole, bisethylhexyloxyphenol methoxyphenyl triazine, bisdisulizole disodium, diometriazole trisiloxane, octyltriazone, iscotrizinol, polysilicone-15, isopentenyl-4-methoxycinnamate, mixtures thereof or the like. Also suitable for use is octocrylene. Amounts of the sunscreen agents when present may generally range from 0.1 to 30%, preferably from 0.5 to 20%, optimally from 0.75 to 10% by weight.

The compositions (e.g., water-in-oil, or oil-in-water or double emulsions or suspensions) suitable for providing improved sunscreen protection typically include cosmetically acceptable carrier components in addition to the non-ionic, branched heteroglycan and sunscreen as described herein. Water, nevertheless, is the most preferred carrier. Amounts of water may range from about 1 to about 98%, and preferably, from about 5 to about 90%, and most preferably, from about 35 to about 80%, and optimally, from about 40 to about 75% by weight, based on total weight of the composition.

Cosmetically acceptable carriers suitable for use in this invention may include mineral oils, silicone oils, synthetic or natural esters, and alcohols. Amounts of these materials may range from about 0.1 to about 50%, and preferably, from about 0.1 to about 30%, and most preferably, from about 1 to about 20% by weight of the composition.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from about 3 to about 9, and preferably, from about 4 to about 5 silicon atoms.

Linear volatile silicone materials generally have viscosities of less than about 5 centistokes at 25°C while cyclic materials typically have viscosities of less than about 10 centistokes.

Nonvolatile silicone oils useful as carrier material include polyalkyl siloxanes, polyalkylaryl siloxanes and polyether siloxane copolymers. The essentially non-volatile polyalkyl siloxanes useful herein include, for example, polydimethylsiloxanes (like dimethicone) with viscosities of from about 5 to about 100,000 centistokes at 25°C. Silicone oils (especially, Dimethicone 35 to 75 centistokes) suitable for use are often made commercially available from Dow Corning are preferred.

Among suitable esters are:
(1) Alkenyl or alkyl esters of fatty acids having 10 to 20 carbon atoms like isopropyl palmitate, isopropyl isostearate, isononyl isonanonoate, oleyl myristate, isopropyl myristate, oleyl stearate, and oleyl oleate;
(2) Ether-esters such as fatty acid esters of ethoxylated fatty alcohols;
(3) Polyhydric alcohol esters such as ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters;
(4) Wax esters such as beeswax, spermaceti, myristyl myristate, stearyl stearate; and
(5) Sterol esters, of which soya sterol and cholesterol fatty acid esters are examples thereof.

Emulsifiers may be present in the composition of the present invention. Total concentration of the emulsifier may range from about 0.1 to about 40%, and preferably, from about 1 to about 20%, and most preferably, from about 1 to about 5% by weight of the composition, including all ranges subsumed therein. The emulsifier may be selected from the group consisting of anionic, nonionic, cationic and amphoteric actives. Particularly preferred nonionic actives are those with a C₁₀-C₂₀ fatty alcohol or acid hydrophobe condensed with from about 2 to about 100 moles of ethylene oxide or propylene oxide per mole of hydrophobe; C₂-C₁₀ alkyl phenols condensed with from 2 to 20 moles of alkylene oxide; mono- and di- fatty acid esters of ethylene glycol; fatty acid monoglyceride; sorbitan, mono- and di- C₈-C₂₀ fatty acids; and polyoxyethylene sorbitan as well as combinations thereof.

Preferred anionic emulsifiers include alkyl ether sulfate and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, alkylethercarboxylates and combinations thereof.

Cationic emulsifiers that may be used include, for example, palmitamidopropyltrimonium chloride, distearyldimonium chloride and mixtures thereof. Useful amphoteric emulsifiers include cocoamidopropyl betaine, C₁₂-C₂₀ trialkyl betaines, sodium lauroamphoacetate, and sodium laurodiamphoacetate or a mixture thereof.

Other generally preferred emulsifiers include glyceryl stearate, glycol stearate, stearamide AMP, PEG-100 stearate, cetyl alcohol as well as emulsifying/thickening additives like hydroxyethylacrylate/sodium acryloyldimethyl taurates copolymer/squalane and mixtures thereof.

Emulsion stabilizers generally classified as vegetable based liquids may also be used. Preferred stabilizers are sold under the name Oilwax LC and made available commercially by Lotioncrafter.

Preservatives can desirably be incorporated into the compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are sodium benzoate, iodopropynyl butyl carbamate, methylisothiazolinone, iodopropynylbutylcarbamate, phenoxyethanol, methyl paraben, propyl paraben, imidazolidinyl urea, sodium dehydroacetate, ethylhexylglycerin and benzyl alcohol and alkane diols. In an especially preferred embodiment, the alkane diols suitable for use are C₆-C₁₂ alkanes that are vicinally substituted with hydroxy groups. Illustrative examples include 1,2-octane diol (caprylyl glycol), 2,3-octane diol, 1,2-nonane diol, 1,2-decane diol, 1,2-hexane diol, 3,4-octane diol, mixtures thereof or the like where caprylyl glycol is typically the most preferred. The preservatives should be selected having regard for the use of the composition and possible incompatibilities between the preservatives and other ingredients in the emulsion. Preservatives are preferably employed in amounts ranging from 0.001% to 5% by weight of the composition, and preferably, from 0.01% to 3%, and most preferably, from 0.02% to 2% by weight of the total weight of the composition including all ranges subsumed therein.

Synthetic polymers are a particularly useful class of effective thickening agents. This category includes crosslinked polyacrylates such as the Carbomers, polyacrylamides such as Sepigel® 305 and taurate copolymers such as Simulgel EG® and Aristoflex® AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100.

Amounts of the thickener, when used, may range from about 0.001 to about 5%, and preferably, from about 0.1 to about 3%, and most preferably, from about 0.2 to about 1.5% by weight of the composition including all ranges subsumed therein.

Conventional humectants may be employed in the present invention. These are generally polyhydric alcohol-type materials. Typical polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. Most preferred is glycerin, propylene glycol or a mixture thereof. The amount of humectant employed may range anywhere from 0.5 to 20%, preferably between 1 and 15% by weight of the composition.

Fragrances, colorants, fixatives and abrasives may optionally be included in compositions of the present invention. Each of these substances may range from about 0.05 to about 5%, preferably between 0.1 and 3% by weight.

Other components suitable for use herein include opacifiers like TiO₂ and ZnO and colorants like iron oxide red, yellow and black. Such opacifiers and colorants typically have a particle size from 50 to 1200 nm, and preferably, from 50 to 350 nm.

To enhance skin moisturization, actives or components classified as cationic ammonium compounds may optionally be used in the compositions of this invention. Such compounds include salts of hydroxypropyltri (C₁-C₃ alkyl) ammonium mono-substituted polyols, dihydroxypropyltri (C₁-C₃ alkyl) ammonium salts, dihydroxypropyldi (C₁-C₃ alkyl) mono(hydroxyethyl) ammonium salts, 2,3-dihydroxypropyl tri(C₁-C₃ alkyl or hydroxalkyl) ammonium salts or mixtures thereof. In a most preferred embodiment and when desired, the cationic ammonium compound employed in this invention is the quaternary ammonium compound 1,2-dihydroxypropyltrimonium chloride. If used, such compounds typically make up from about 0.01 to about 30%, and preferably, from about 0.1 to about 15% by weight of the composition.

When cationic ammonium compounds are used, preferred additional active for use with the same are moisturizing agents such as substituted ureas like hydroxymethyl urea, hydroxyethyl urea, hydroxypropyl urea; bis(hydroxymethyl) urea; bis(hydroxyethyl) urea; bis(hydroxypropyl) urea; N,N'-dihydroxymethyl urea; N,N'-di-hydroxyethyl urea; N,N'-dihydroxypropyl urea; N,N,N'-tri-hydroxyethyl urea; tetra(hydroxymethyl) urea; tetra(hydroxyethyl) urea; tetra(hydroxypropyl) urea; N-methyl-N'-hydroxyethyl urea; N-ethyl-N,N-N'-hydroxyethyl urea; N-hydroxypropyl-N'-hydroxyethyl urea and N,N'-dimethyl-N-hydroxyethyl urea or mixtures thereof. Where the term hydroxypropyl appears, the meaning is generic for either 3-hydroxy-n-propyl, 2-hydroxy-n-propyl, 3-hydroxy-i-propyl or 2-hydroxy-i-propyl radicals. Most preferred is hydroxyethyl urea. The latter is available as a 50% aqueous liquid from the National Starch & Chemical Division of ICI under the trademark Hydrovance.

Amounts of substituted urea, when used, in the composition of this invention range from about 0.01 to about 20%, and preferably, from about 0.5 to about 15%, and most preferably, from about 2 to about 10% based on total weight of the composition and including all ranges subsumed therein.

When cationic ammonium compound and substituted urea are used, in a most especially preferred embodiment at least from about 1 to about 15% glycerin external to the particle is used, based on total weight of the composition and including all ranges subsumed therein.

Compositions of the present invention may include vitamins as the desired active. Illustrative vitamins are Vitamin A (retinol) as well as retinol esters like retinol palmitate and retinol propionate, Vitamin B₂, Vitamin B₃ (niacinamide), Vitamin B₆, Vitamin C, Vitamin E, Folic Acid and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present in compositions according to the present invention may range from 0.001 to 10%, preferably from 0.01% to 1%, optimally from 0.1 to 0.5% by weight of the composition.

Octadecenedioic acid, azelaic acid, ubiquinone, dihydroxyacetone (DHA) and mixtures thereof may also be used as actives in the composition of this invention. Such compounds, when used, typically make up from about 0.2 to 4.5%, and preferably, from about 0.5 to 3% by weight of the composition, including all ranges subsumed therein.

Other optional actives suitable for use in this invention include resveratrol, resorcinols like 4-ethyl resorcinol, 4-butyl resorcinol, 4-hexyl resorcinol, dimethoxytoluyl propyl resorcinol, 4-cyclopentyl resorcinol, 4-cyclohexylresorcinol, 4-isopropyl resorcinol, alpha-an/or beta-hydroxyacids, petroselinic acid, conjugated linoleic acid, octadecanoic acid, 4-phenylethyl resorcinol (Symwhite 377 from Symrise), undecylenol phenylalanine (Sepiwhite from Seppic) mixtures thereof or the like. Such actives, when used, collectively make up from about 0.0001 to about 12% by weight of the composition. In a preferred embodiment, resorcinol, when used, make up from 0.0001 to 8, and preferably, from 0.0001 to 6, and most preferably, from 0.01 to 4% by weight of the composition.

Desquamation promoters may be present. Illustrative are the alpha-hydroxycarboxylic acids, beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic and its derivatives, lactic and malic acids. Salicylic acid is representative of the beta-hydroxycarboxylic acids. Amounts of these materials when present may range from about 0.01 to about 15% by weight of the composition.

A variety of herbal extracts may optionally be included as actives in compositions of this invention. The extracts may either be water soluble or water-insoluble carried in a solvent which respectively is hydrophilic or hydrophobic. Water and ethanol are the preferred extract solvents. Illustrative extracts include those from green tea, yarrow, chamomile (especially CO₂ derived chamomile), licorice, aloe vera, grape seed, citrus unshui, willow bark, sage, thyme and rosemary. Soy extracts may be used and especially when it is desirable to include retinol.

Also optionally suitable for use include materials like chelators (e.g., EDTA), lipoic acid, retinoxytrimethylsilane (available from Clariant Corp. under the Silcare 1M-75 trademark), dehydroepiandrosterone (DHEA) and combinations thereof. Ceramides (including Ceramide 1, Ceramide 3, Ceramide 3B and Ceramide 6) as well as pseudoceramides may also be useful. Occlusives like Oilwax LC are often desired as is 12-hydroxystearic acid as a skin lightener. Amounts of these materials may range from about 0.000001 to about 10%, preferably from about 0.0001 to about 4%, and most preferably, form 0.001 to 3% by weight of the composition.

Conventional buffers/pH modifiers may be used. These include commonly employed additives like sodium hydroxide, potassium hydroxide, hydrochloric acid, citric acid and citrate/citric acid buffers. In an especially preferred embodiment, the pH of the composition of this invention is from about 4 to about 8, and preferably, from about 4.25 to about 7.75, and most preferably, from about 6 to about 7.5, including all ranges subsumed therein. The composition of this invention may be a solid stick or bar. Viscosity of the composition of this invention is, however, preferably from about 1,000 to about 120,000 cps, and most preferably, from about 5,000 to 80,000 cps, taken at ambient temperature NS and a shear rate of 1s⁻¹ with a strain controlled parallel plate rheometer made commercially available from suppliers like T.A. Instruments under the Ares name.

In the present invention, compositions with polymers of D-glucose and/or non-ionic, branched heteroglycan and sunscreen are desired including compositions comprising locust bean gum, avobenzone, octylmethoxycinnamate with or without an alkandiol like caprylyl glycol; and compositions comprising lotus bean gum, avobenzone, octylmethoxycinnamate, and ensulizole with or without an alkanediol like caprylyl glycol; and compositions comprising lotus bean gum, avobenzone and octylmethoxycinnamate with or without an alkanediol like caprylyl glycol. Other desired compositions include those comprising octylmethoxycinnamate, avobenzone and beta oat glucan, and ethylhexylsalicylate, ensulizole and beta oat glucan.

A wide variety of packaging can be employed to store and deliver the composition of this invention. Packaging is often dependent upon the type of personal care end-use. For instance, leave-on skin lotions and creams, shampoos, conditioners and shower gels generally employ plastic containers with an opening at a dispensing end covered by a closure. Typical closures are screw-caps, non-aerosol pumps and flip-top hinged lids. Packaging for antiperspirants, deodorants and depilatories may involve a container with a roll-on ball on a dispensing end. Alternatively these types of personal care products may be delivered in a stick composition formulation in a container with propel-repel mechanism where the stick moves on a platform towards a dispensing orifice. Metallic cans pressurized by a propellant and having a spray nozzle serve as packaging for antiperspirants, shave creams and other personal care products. Toilette bars may have packaging constituted by a cellulosic or plastic wrapper or within a cardboard box or even encompassed by a shrink wrap plastic film.

The following examples are provided to facilitate an understanding of the present invention. The examples are not intended to limit the scope of the claims.

### Examples

The formulations in Table 1 were made by combining the indicated sunscreen and saccharide ingredients into the base formulas of either Table 2 or Table 3 and the SPF and UVAPF properties were measured using the methods described below.

Examples 1 to 12 used the base formula of Table 2 and examples 13 to 16 used the base formula of Table 3. The formulations in Table 1 were assessed for in vitro sunscreen benefits utilizing a commercially available Optometrics 290S SPF meter (Optometrics Inc., Littleton, MA). Each formulation was separately applied at a dosage of 2mg/cm² on a PMMA plate (7 cm²) and allowed to air dry for about 20 minutes. Average values were obtained for six (6) readings per plate.

**TABLE 1**

| Example Number | Sunscreen(s) | Saccharide(s) | Inventive/ Comparative (I)/(C) | In Vitro SPF (2mg/cm²) | |
|---|---|---|---|---|---|
| | | | | SPF | UVAPF |
| 1 | Octylmethoxycinnamate, 3 wt. % | None | Control | 18 | 10 |
| | Avobenzene, 1.5 wt. % | | | | |
| 2 | Octylmethoxycinnamate, 3 wt. % | Oligoquat M (1), 2 wt. % | C | 20 | 13 |
| | Avobenzene, 1.5 wt. % | | | | |
| 3 | Octylmethoxycinnamate, 3 wt. % | Hydroxy propyl Cellulose, 2 wt. % | C | 21 | 11 |
| | Avobenzene, 1.5 wt. % | | | | |
| 4 | Octylmethoxycinnamate, 3 wt. % | Locust Bean Gum (2), 2 wt. % | I | 32 | 19 |
| | Avobenzene, 1.5 wt. % | | | | |
| 5 | Avobenzene, 1.5 wt. % | None | Control | 10 | 9 |
| | Octocrylene 3 wt. % | | | | |
| 6 | Octylmethoxycinnamate, 3 wt. % | Beta oat glucan (3), 2 wt. % | I | 37 | 23 |
| | Avobenzene, 1.5 wt. % | | | | |
| 7 | Octylmethoxycinnamate, 3 wt. % | Xilogel (4), 2 wt. % | I | 41 | 24 |
| | Avobenzene, 1.5 wt. % | | | | |
| 8 | Octylmethoxycinnamate, 3 wt. % | Pullulan (5), 2 wt. % | I | 44 | 27 |
| | Avobenzene, 1.5 wt. % | | | | |
| 9 | Octylmethoxycinnamate, 3 wt. % | Pullulan (5), 2 wt. % | I | 51 | 31.5 |
| | Avobenzene, 1.5 wt. % | Caprylyl Glycol, 2 wt. % | | | |
| 10 | Octocrylene, 3 wt. % | None | Control | 10 | 9 |
| | Avobenzene, 1.5 wt. % | | | | |
| 11 | Octocrylene, 3 wt. % | Pullulan (5), 2 wt. % | I | 16 | 10.4 |
| | Avobenzene, 1.5 wt. % | | | | |
| 12 | Octocrylene, 3 wt. % | Pullulan (5), 2 wt. % Caprylyl Glycol, 2 wt. % | I | 28.3 | 25 |
| | Avobenzene, 1.5 wt. % | | | | |
| 13 | Avobenzene, 3 wt. % | None | Control | 28 | 19 |
| | Ensulizole, 3 wt. % | | | | |
| | EthylHexyl Salicylate, 5 wt. % | | | | |
| 14 | Avobenzene, 3 wt. % | Hydroxypropyl cellulose, 2 wt. % | C | 20 | 12 |
| | Ensulizole, 3 wt. % | | | | |
| | Ethylhexyl Salicylate, 5 wt. % | | | | |
| 15 | Avobenzene, 3 wt. % | Beta oat glucan (3), 2 wt. % | I | 53 | 40 |
| | Ensulizole, 3 wt. % | | | | |
| | Ethylhexyl Salicylate, 5 wt. % | | | | |
| 16 | Avobenzene, 3 wt. % | Xilogel (4), 2 wt. % | I | 62 | 50 |
| | Ensulizole, 3 wt. % | | | | |
| | Ethylhexyl Salicylate, 5 wt. % | | | | |

| | | | | | |
|---|---|---|---|---|---|
| (1) stearyl dihydroxypropyldimonium oligosaccharide (2) galactomannan (3) D-glucose polysaccharide (4) Xylose glucan (5) Maltotriose Avobenzone (Parsol 1789, butyl methoxydibenzoylmethane) Ensulizole (Phenylbenzimidazole Sulfonic Acid) | | | | | |

**Table 2 (Stearic acid base)**

| MATERIAL | % w/w |
|---|---|
| | |
| Stearic Acid | 17 |
| Cetyl Alcohol | 0.53 |
| Methyl Paraben | 0.2 |
| Glycerin | 1.0 |
| Potassium Hydroxide (KOH, 50%) | 0.96 |
| Disodium EDTA | 0.04 |
| Dimethicone | 0.5 |
| Propyl Paraben | 0.1 |
| Isopropyl Myristate | 0.75 |
| Niacinamide | 1.25 |
| Phenoxyethanol | 0.4 |
| TiO2 | 0.9 |
| DI Water | q.s |

**Table 3 (Glycol stearate base)**

| MATERIAL | % w/w |
|---|---|
| | |
| | |
| Methylparaben | 0.2 |
| Propylparaben | 0.1 |
| Disodium EDTA | 0.1 |
| Carbopol Ultrez 10 | 0.4 |
| Glycerin | 2.5 |
| Xanthan Gum | 0.5 |
| Niacinamide | 0.1 |
| Potassium Hydroxide (50%) | 0.9 |
| Sodium Hydroxide (40%) | 0.5 |
| Stearic Acid | 2.4 |
| Glycol Stearate/Stearamide AMP | 1.4 |
| Glyceryl Monostearate | 0.647 |
| Cetyl Alcohol | 0.4 |
| PEG-100 Stearate | 1.2 |
| Dimethicone | 1 |
| Phenoxyethanol | 0.7 |
| | |
| | |
| DI Water | q.s |

The results above unexpectedly show that compositions made consistent with this invention yield an increase in sun protection factors compared to both a control without saccharide and a comparative saccharide i.e hydroxypropyl cellulose.

## Claims

1. A composition comprising:
(a) 0.5 to 7 wt. % of polysaccharide(s) selected from a water soluble and/or water dispersible nonionic polymer(s) of D-glucose, water soluble nonionic, branched heteroglycan(s) or a blend thereof;
(b) 0.1 to 30 wt. % of a sunscreen;
(c) 0.1 to 10 wt. % of a C3 to C16 alkane, mono alkoxy or polyalkoxy diol(s) or a blend thereof, optionally wherein the diol has vicinally substituted hydroxyl groups;
(d) wherein the ratio of polysaccharide(s) to alkane/alkoxy diol(s) is in the range of 70:1 to 1:40; and
(e) a cosmetically acceptable carrier;
wherein the alkane diol includes 1,2-octane diol.

2. The composition according to claim 1 wherein the composition further comprises phenoxyethanol.

3. The composition according to claims 1 or 2 wherein the polysaccharide(s) includes a polymer of D-glucose with a number average molecular weight from 50,000 to 4,500,000.

4. The composition according to any one of claims 1 to 3 wherein the polysaccharide(s) includes a nonionic, branched heteroglycan and comprises backbone groups represented as having repeat units of I and II, or II 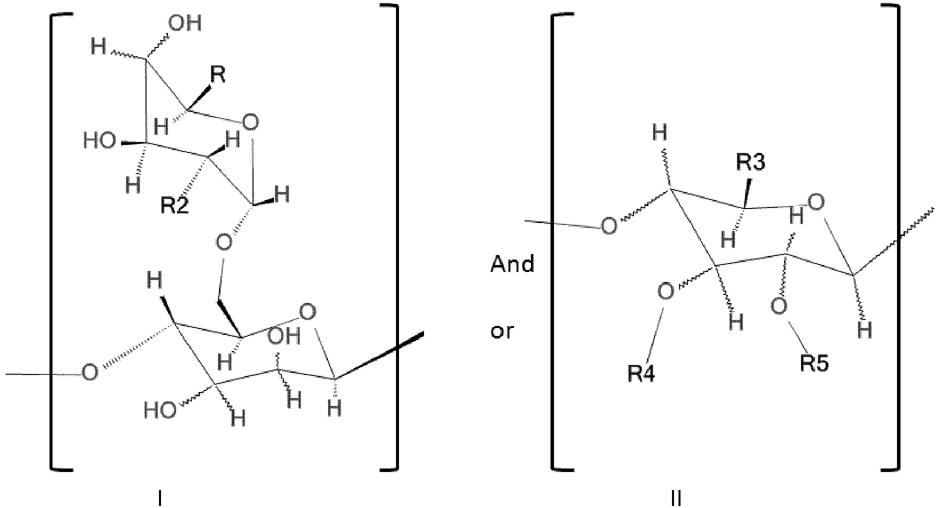 wherein
R= H, or CH₂OH;
R2= OH, or C₆ monosaccharide;
R3= H, CH₂OH,or acetate;
R4= H, or [C6 monosaccharide]ₙ n= integer from approximately 200 to 25,000;
R5= H, or C₅ monosaccharide;
and/or agar, dextran, pullulan, arabinogalactane or blends thereof.

5. The composition according to claim 4 wherein the non-ionic, branched heteroglycan comprises backbone groups that are at least 85% mannose group and further wherein the heteroglycan comprises branched groups that are at least 95% galactose group.

6. The composition according to claims 4 or 5 wherein the non-ionic, branched heteroglycan is derived from guar, tara, fenugreek, locust bean or carob gum or a mixture thereof.

7. The composition according to any one of claims 1 to 6 wherein the sunscreen is octylmethoxycinnamate, ethylhexyl salicylate, phenylbenzimidazole sulfonic acid, ethylhexyl p-methoxycinnamate, butyl methoxydibenzoylmethane, benzophenone-3, titanium dioxide, zinc oxide, p-aminobenzoic acid, octyldimethyl-PABA, 2-ethoxyethyl p-methoxy cinnamate, benzophenone-1, benzophenone-2, benzophenone-6, benzophenone-8, benzophenone-9, benzophenone-12, homomethyl salicylate, menthyl anthranilate, benzophenone-4, triethanolamine salicylate, terephthalylidene dicamphor sulfonic acid, bisoctriazole, bisethylhexyloxyphenol methoxyphenyl triazine, bisdisulizole disodium, diometriazole trisiloxane, octyltriazone, iscotrizinol, polysilicone-15, isopentenyl-4-methoxycinnamate, octocrylene or a mixture thereof.

8. The composition according to claim 7 wherein the sunscreen is octylmethoxycinnamate, ethylhexyl salicylate, phenylbenzimidazole sulfonic acid, ethylhexyl p-methoxycinnamate, octocrylene, butyl methoxydibenzoylmethane or a mixture thereof.

9. The composition according to any one of claims 1 to 8 wherein the composition imparts an improved SPF and UVA-PF upon use compared to the same composition absent 0.5 to 7 wt. % of polysaccharide(s) selected from a water soluble or dispersible nonionic polymer(s) of D-glucose, water soluble or dispersible nonionic, branched heteroglycan(s) or a blend thereof.

10. Composition according to any one of claims 1 to 9 for use in a method for protecting skin from the sun, **characterised in that** the composition is applied to the skin.

11. A composition comprising:
(a) 0.5 to 7 wt. % of polysaccharide(s) selected from locust bean gum, tara gum, pullulan, beta oat glucan, xylose glucan, larch arabinogalactane or a blend thereof;
(b) about 0.1 to 30 wt. % of a sunscreen; and
(e) a cosmetically acceptable carrier
wherein the sunscreen is a mixture of two or more of octylmethoxycinnamate, ethylhexyl salicylate, phenylbenzimidazole sulfonic acid, ethylhexyl p-methoxycinnamate, octocrylene and butyl methoxydibenzoylmethane.

12. The composition according to claim 11 wherein the composition imparts an improved SPF and UVA-PF upon use compared to the same composition absent 0.5 to 7 wt. % of polysaccharide(s) selected from a water soluble nonionic polymer(s) of D-glucose, water soluble nonionic, branched heteroglycan(s) or a blend thereof.

13. Composition according to claims 11 or 12 for use in a method for protecting skin from the sun, **characterised in that** the composition is applied to the skin.

## Patentansprüche

1. Zusammensetzung, umfassend:
(a) 0,5 bis 7 Gew.-% Polysaccharid(e), ausgewählt aus einem wasserlöslichen und/oder in Wasser dispergierbaren nichtionischen Polymer(en) von D-Glucose, wasserlöslichen nichtionischen verzweigten Heteroglycan(en) oder einer Mischung davon;
(b) 0,1 bis 30 Gew.-% eines Sonnenschutzmittels;
(c) 0,1 bis 10 Gew.-% von einem C3- bis C16-Alkan, Monoalkoxy- oder Polyalkoxydiol(en) oder einer Mischung davon, wobei das Diol gegebenenfalls vicinal substituierte Hydroxylgruppen aufweist;
(d) wobei das Verhältnis von Polysaccharid(en) zu Alkan/Alkoxydiol(en) im Bereich von 70:1 bis 1:40 liegt; und
(e) einen kosmetisch akzeptablen Träger;
wobei das Alkandiol 1,2-Octandiol umfasst.

2. Zusammensetzung nach Anspruch 1, wobei die Zusammensetzung ferner Phenoxyethanol umfasst.

3. Zusammensetzung nach den Ansprüchen 1 oder 2, wobei das bzw. die Polysaccharid(e)ein Polymervon D-Glucose mit einem Zahlenmittel des Molekulargewichts von 50.000 bis 4.500.000 enthält.

4. Zusammensetzung nach irgendeinem der Ansprüche 1 bis 3, wobei das bzw. die Polysaccharid(e) ein nichtionisches verzweigtes Heteroglycan und umfassend Grundgerüstgruppen, die so dargestellt sind, dass sie Wiederholungseinheiten von I und II oder II aufweisen 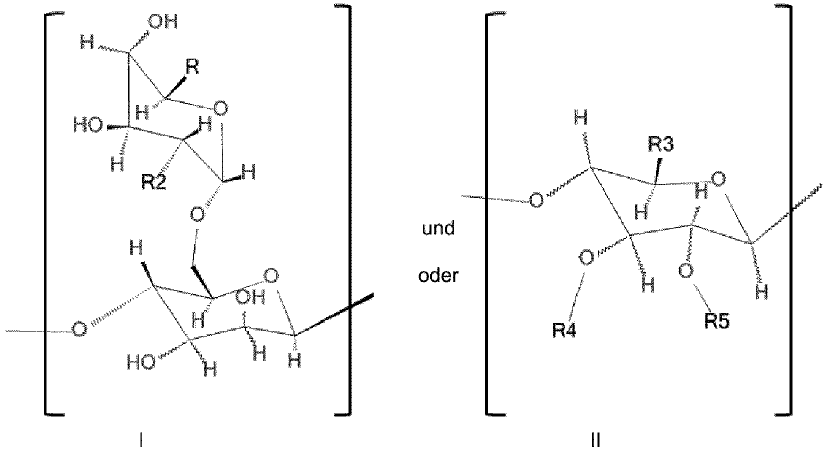 worin
R = H oder CH₂OH;
R2 = OH oder C₆-Monosaccharid;
R3 = H, CH₂OH oder Acetat;
R4 = H oder [C₆-Monosaccharid]ₙ n = ganze Zahl von etwa 200 bis 25.000;
R5 = H oder Cs-Monosaccharid;
und/oder Agar, Dextran, Pullulan, Arabinogalactan oder Mischungen davon beinhalten.

5. Zusammensetzung nach Anspruch 4, wobei das nichtion ische verzweigte Heteroglycan Grundgerüstgruppen umfasst, die mindestens 85% Mannosegruppe sind, und ferner wobei das Heteroglycan verzweigte Gruppen umfasst, die mindestens 95% Galactosegruppe sind.

6. Zusammensetzung nach den Ansprüchen 4 oder 5, wobei das nichtionische verzweigte Heteroglycan aus Guar, Tara, Bockshornklee, Johannisbrot oder Johannisbrotkernmehl oder einer Mischung davon abgeleitet ist.

7. Zusammensetzung nach irgendeinem der Ansprüche 1 bis 6, wobei das Sonnenschutzmittel Octylmethoxycinnamat, Ethylhexylsalicylat, Phenylbenzimidazolsulfonsäure, Ethylhexyl-p-methoxycinnamat, Butylmethoxydibenzoylmethan, Benzophenon-3, Titandioxid, Zinkoxid, p-Aminobenzoesäure, Octyldimethyl-PABA, 2-Ethoxyethyl-p-methoxycinnamat, Benzophenon-1, Benzophenon-2, Benzophenon-6, Benzophenon-8, Benzophenon-9, Benzophenon-12, Homomethylsalicylat, Menthylanthranilat, Benzophenon-4,Triethanolaminsalicylat, Tetraphthalylidendicamphersulfonsäure, Bisoctriazol, Bisethylhexyloxyphenolmethoxyphenyltriazin, Bisdisulizoldinatrium, Diometriazoltrisiloxan, Octyltriazon, Iscotrizinol, Polysilicon-15, Isopentenyl-4-methoxycinnamat, Octocrylen oder eine Mischung davon ist.

8. Zusammensetzung nach Anspruch 7, worin das Sonnenschutzmittel Octylmethoxycinnamat, Ethylhexylsalicylat, Phenylbenzimidazolsulfonsäure, Ethylhexyl-p-methoxycinnamat, Octocrylen, Butylmethoxydibenzoylmethan oder eine Mischung davon ist.

9. Zusammensetzung nach irgendeinem der Ansprüche 1 bis 8, wobei die Zusammensetzung bei Verwendung einen verbesserten SPF und UVA-PF im Vergleich zu derselben Zusammensetzung verleiht, der 0,5 bis 7 Gew.-% Polysaccharid(e), ausgewählt aus einem in Wasser löslichen oder dispergierbaren nichtionischen Polymer(en) von D-Glucose, in Wasser löslichen oderdispergierbaren nichtionischen verzweigten Heteroglycan(en) oder einer Mischung davon, fehlen.

10. Zusammensetzung nach irgendeinem der Ansprüche 1 bis 9 zur Verwendung in einem Verfahren zum Schutz der Haut vor der Sonne, **dadurch gekennzeichnet, dass** die Zusammensetzung auf die Haut aufgebracht wird.

11. Zusammensetzung, umfassend:
(a) 0,5 bis 7 Gew.-% Polysaccharid(e), ausgewählt aus Johannisbrotkernmehl, Taragummi, Pullulan, Beta-Glucan aus Hafer, Xyloseglucan, Arabinogalactan aus Lärchen oder einer Mischung davon;
(b) etwa 0,1 bis 30 Gew.-% eines Sonnenschutzmittels; und
(e) einen kosmetisch akzeptablen Träger,
wobei das Sonnenschutzmittel eine Mischung von zwei oder mehr aus Octylmethoxycinnamat, Ethylhexylsalicylat, Phenylbenzimidazolsulfonsäure, Ethylhexyl-p-methoxycinnamat, Octocrylen und Butylmethoxydibenzoylmethan ist.

12. Zusammensetzung nach Anspruch 11, wobei die Zusammensetzung bei Verwendung einen verbesserten LSF und UVA-PF im Vergleich zu derselben Zusammensetzung verleiht, der 0,5 bis 7 Gew.-% Polysaccharid(e), ausgewählt aus einem wasserlöslichen nichtionischen Polymer(en) von D-Glucose, wasserlöslichen nichtionischen verzweigten Heteroglycan(en) oder einer Mischung davon, fehlen.

13. Zusammensetzung nach den Ansprüchen 11 oder 12 zur Verwendung in einem Verfahren zum Schutz der Haut vor der Sonne, **dadurch gekennzeichnet, dass** die Zusammensetzung auf die Haut aufgebracht wird.

## Revendications

1. Composition comprenant :
(a) 0,5 à 7 % en poids de polysaccharide(s) choisis parmi un ou plusieurs polymères de D-glucose solubles dans l'eau et/ou dispersibles dans l'eau, non ioniques, hétéroglycanes ramifiés solubles dans l'eau, non ioniques, ou un mélange de ceux-ci ;
(b) 0,1 à 30 % en poids d'un écran solaire ;
(c) 0,1 à 10 % en poids d'un alcane en C3 à C16, de monoalcoxy ou polyalcoxydiol(s) ou un mélange de ceux-ci, facultativement dans laquelle le diol comporte des groupes hydroxyle vicinalement substitués ;
(d) dans laquelle le rapport du ou des polysaccharide(s) au(x) alcane/alcoxy diol(s) est dans la plage de 70:1 à 1:40 ; et
(e) un véhicule acceptable en cosmétique ;
dans laquelle l'alcanediol comprend le 1,2-octanediol.

2. Composition selon la revendication 1 où la composition comprend en outre du phénoxyéthanol.

3. Composition selon les revendications 1 ou 2 dans laquelle le(s) polysaccharide(s) comprennent un polymère de D-glucose ayant un poids moléculaire moyen en nombre de 50 000 à 4 500 000.

4. Composition selon l'une quelconque des revendications 1 à 3 dans laquelle le(s) polysaccharide(s) comprennent un hétéroglycane ramifié, non ionique et comprend des groupes de chaîne principale représentés comme ayant des motifs de répétition de I et II, ou II 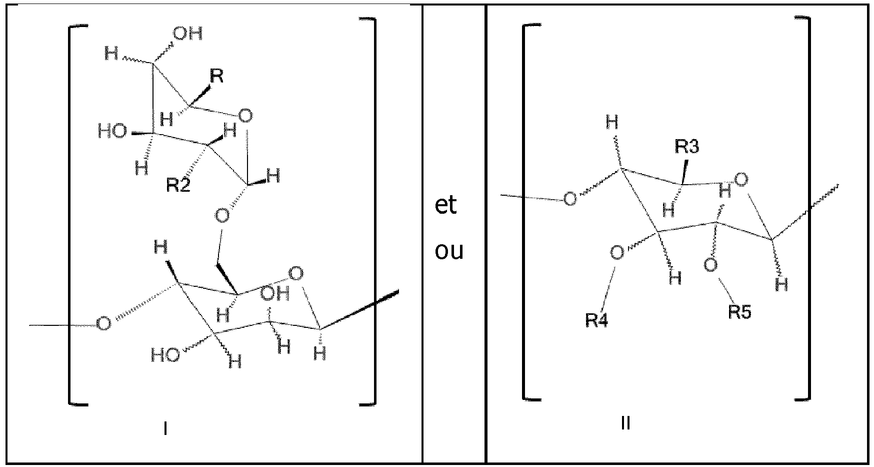 dans lesquels
R = H ou CH₂OH ;
R2 = OH ou monosaccharide en C₆ ;
R3 = H, CH₂OH ou acétate ;
R4 = H ou [C6 monosaccharide]ₙ alcane diol = entier d'approximativement 200 à 25 000 ;
R5 = H ou monosaccharide en C₅ ;
et/ou le gélose, le dextrane, le pullulane, l'arabinogalactane ou des mélanges de ceux-ci.

5. Composition selon la revendication 4 dans laquelle l'hétéroglycane ramifié, non-ionique comprend des groupes de chaîne principale qui sont au moins 85 % de groupe mannose et en outre dans laquelle l'hétéroglycane comprend des groupes ramifiés qui sont au moins 95 % de groupe galactose.

6. Composition selon les revendications 4 ou 5 dans laquelle l'hétéroglycane ramifié, non ionique est dérivé de guar, tara, fenugrec, caroube ou gomme de caroube ou un mélange de ceux-ci.

7. Composition selon l'une quelconque des revendications 1 à 6 dans laquelle l'écran solaire est le méthoxycinnamate d'octyle, le salicylate d'éthylhexyle, l'acide phénylbenzimidazole-sulfonique, le p-méthoxycinnamate d'éthylhexyle, le butylméthoxydibenzoylméthane, la benzophénone-3, le dioxyde de titane, l'oxyde de zinc, l'acide p-aminobenzoïque, octyldiméthyl-PABA, le 2-éthoxyéthyle le p-méthoxycinnamate, la benzophénone-1, la benzophénone-2, la benzophénone-6, la benzophénone-8, la benzophénone-9, la benzophénone-12, le salicylate d'homométhyle, l'anthranilate de menthyle, la benzophénone-4, le salicylate de triéthanolamine, l'acide téréphtalylidène-dicamphre-sulfonique, le bisoctriazole, la biséthylhexyloxyphénol-méthoxyphényl-triazine, le bisdisulizole disodique, le diométriazole-trisiloxane, l'octyltriazone, l'iscotrizinol, la polysilicone-15, l'isopentényl-4-méthoxycinnamate, l'octocrylène ou un mélange de ceux-ci.

8. Composition selon la revendication 7 dans laquelle l'écran solaire est le méthoxycinnamate d'octyle, le salicylate d'éthylhexyle, l'acide phénylbenzimidazole-sulfonique, le p-méthoxycinnamate d'éthylhexyle, l'octocrylène, le butylméthoxydibenzoylméthane ou un mélange de ceux-ci.

9. Composition selon l'une quelconque des revendications 1 à 8 où la composition confère un SPF et un UVA-PF améliorés lors de l'utilisation par rapport à la même composition en l'absence de 0,5 à 7 % en poids de polysaccharide(s) choisi(s) parmi un ou plusieurs polymère(s) de D-glucose solubles dans l'eau, non ioniques, hétéroglycanes ramifiés solubles dans l'eau, non ioniques, ou un mélange de ceux-ci.

10. Composition selon l'une quelconque des revendications 1 à 9 pour utilisation dans un procédé de protection de la peau contre le soleil, **caractérisée en ce que** la composition est appliquée sur la peau.

11. Composition comprenant :
(a) 0,5 à 7 % en poids de polysaccharide(s) choisi(s) parmi la gomme de caroube, la gomme tara, le pullulane, le bêta-glucane d'avoine, le xylose glucane, l'arabinogalactane de mélèze ou un mélange de ceux-ci ;
(b) environ 0,1 à 30 % en poids d'un écran solaire ; et
(c) un véhicule acceptable en cosmétique
dans laquelle l'écran solaire est un mélange de deux ou plus parmi le méthoxycinnamate d'octyle, le salicylate d'éthylhexyle, l'acide phénylbenzimidazole-sulfonique, le p-méthoxycinnamate d'éthylhexyle, l'octocrylène et le butylméthoxydibenzoylméthane.

12. Composition selon la revendication 11 où la composition confère un SPF et un UVA-PF améliorés lors de l'utilisation par rapport à la même composition en l'absence de 0,5 à 7 % en poids de polysaccharide(s) choisi(s) parmi un ou plusieurs polymère(s) de D-glucose solubles dans l'eau, non ioniques, hétéroglycanes ramifiés solubles dans l'eau, non ioniques, ou un mélange de ceux-ci.

13. Composition selon les revendications 11 ou 12 pour utilisation dans un procédé pour protéger la peau du soleil, **caractérisée en ce que** la composition est appliquée sur la peau.
